# EUROPEAN PATENT APPLICATION

(11) **EP 4 032 975 A1**
(43) Date of publication of application: **27.07.2022**
(21) Application number: 20864051.6
(22) Date of filing: 26.05.2020
(51) Int. Cl.: C12N 5/10, C12N 15/62, C12N 15/867, A61K 39/00, A61P 35/00

(54) **ANTI CD70 CAR-T CELL, AND PREPARATION METHOD THEREFOR AND USE THEREOF**

(30) Priority: 11.09.2019 CN 201910857176
(71) Applicant: ZHEJIANG BLUE SHIELD PHARMACEUTICAL CO., LTD., Keqiao Economic Development Zone, Keqiao District Shaoxing Zhejiang 312030 (CN)
(72) Inventor: GUO, Zhigang, Shaoxing, Zhejiang 312030 (CN); JI, Feng, Shaoxing, Zhejiang 312030 (CN); LIU, Rui, Shaoxing, Zhejiang 312030 (CN); WU, Ting, Shaoxing, Zhejiang 312030 (CN); LI, Enjie, Shaoxing, Zhejiang 312030 (CN); LI, Lulu, Shaoxing, Zhejiang 312030 (CN); LI, Menghan, Shaoxing, Zhejiang 312030 (CN); HU, Zhigang, Shaoxing, Zhejiang 312030 (CN)
(74) Representative: Cabinet Laurent & Charras
(86) International application number: PCT/CN2020/092192
(87) International publication number: WO 2021/047208

(57) **Abstract**

Provided are a CAR-T cell specifically recognizing CD70, and a preparation method therefor. The method comprises expressing a SCFV(Anti CD70)-CD8^{™}-4-1BB-CD3ζ molecule in a T cell. The CAR-T cell has tumor killing activity and may be used for preparing a medication in the treatment of a tumor with the high expression level of CD70 molecules.

## Description

### Technical field

The invention belongs to the field of molecular biology and cell biology, in particular to a CAR-T cell specifically recognizing CD70 and a preparation method thereof, as well as its use in drugs for treating diseases related to high expression of CD70 molecules.

### Background technology

The low immune system function of cancer patients caused by radiotherapy and chemotherapy is an important reason for the recurrence of cancer, and it is also a major problem that plagues the medical community. Chimeric antigen receptor T-cell immuno-therapy (CAR-T) has become the hotspot of global biotechnology competition because of its unique anti-tumor advantages. CAR-T cells are obtained by coupling the single-chain variable region (SCFV) of an antibody which can recognize a certain tumor antigen with the intracellular region of CD8^{™}, 4-1BB, CD3-ζ chain, etc., to form a chimeric protein, which is transfected into T cells of a patient *in vitro* by gene transduction to express chimeric antibody receptor (CAR). After the patient's T cells are "reprogrammed", a large number of killing CAR-T cells are generated, which can specifically target tumor cells and will not harm normal cells. Because it induces and activates autologous cells, this therapy has no usual immune rejection and no drug resistance in traditional treatment.

A CAR-T cell is formed by connecting the single chain variable region of a monoclonal antibody with the signal transduction region of a T cell. After the antibody binds to the corresponding tumor antigen, it can activate T cells in a non-restrictive manner by the major histocompatibility complex, and then exert anti-tumor effect. The structure of CAR is divided into an extracellular antigen binding region, a hinge region, a transmembrane region and an intracellular signal region. The selection of target antigens is a key determinant of the specificity and effectiveness of CAR and the safety of genetically modified T cells. The extracellular antigen binding region: it is usually formed by linking light and heavy chains of monoclonal antibodies against tumor-associated antigens. The hinge region: for epitopes far away from cell membrane, CAR-T cells without hinge region can recognize and bind to antigens; for epitopes at the proximal end of the membrane, a flexible hinge region is necessary for antigen recognition. Therefore, it is necessary to adjust the length of the hinge region for different tumor-associated antigens to exert the targeted binding ability well. The transmembrane region: the transmembrane regions derived from CD3, CD8 and CD8 are commonly used to construct CAR, and play an important role in dimerization of CAR and T cell activation. The intracellular signal region: it is composed of co-stimulatory molecules such as CD8, CD134 and CD137, in which CD8 can recruit PI3K, Grb2 and other molecules to regulate the activity of key transcription factors.

CD134 can promote the proliferation of T cells *in vitro* and increase the secretion of IL-2. CD137 is a tumor necrosis factor family receptor, which can activate the downstream JNK, p38, MAPK and INF-κ B signaling pathways. Most researchers have fused the co-stimulatory signal molecules of CD8 or CD137 to the upstream of CD3 domain. According to the different compositions of intracellular signal transduction regions, CAR-T cells can be divided into three generations. The intracellular part of the first generation CAR-T cells only contains CD3ζ domain, which can induce T cell activation and initial cytotoxicity, but the survival time of them is short and there is little or no IL-2 production; the second generation CAR-T cells fuse the intracellular part of co-stimulatory molecules such as CD8 and CD137 to the upstream of CD3 domain, and the effect is obviously enhanced compared with the first generation, so it is widely used; the third generation CAR-T cells fuse two co-stimulatory molecules to the upstream of CD3 domain at the same time, and their anti-tumor activity is still inconclusive compared with the second generation CAR-T cells. CAR-T cells play an important role in the immunotherapy of hematological tumors, especially CD19-CAR-T cells in the treatment of lymphocytic tumors, and some of them have entered Phase II clinical trials. Most of CAR-T cell studies in solid tumors are still in the stage of animal experiments, but some small samples of clinical trials and case reports have achieved good results.

Renal cell carcinoma (renal cancer for short) is a highly malignant tumor in the urinary system and one of the most common tumors. It is a malignant tumor that originates from the urinary tubule epithelial system of the renal parenchyma, also known as renal adenocarcinoma, accounting for 80%~90% of renal malignant tumors. According to the investigation, renal cancer occupies the second place in genitourinary system tumors in China, only next to bladder tumors, accounting for 2%~3% of adult malignant tumors and about 20% of children malignant tumors. There are obvious differences in the incidence between male and female. According to statistics, the ratio of male to female is 2: 1. The incidence of renal cancer increases with age. Some data show that the high incidence age of renal cancer is 40~55 years old. The incidence of renal cancer in China is increasing year by year, ranking 10th in the incidence of male malignant tumors.

CD70 is a member of tumor necrosis factor receptor (TNFR) superfamily, which has the ability to regulate the activation, proliferation and differentiation of T and B cells, and plays an important role in maintaining immune response. At the same time, under physiological conditions, CD70 is only temporarily expressed in activated lymphocytes, but it is highly expressed in 38%~68% of renal clear cell carcinoma cases and 30%~60% of renal papillary cell carcinoma cases. Moreover, CD70 is expressed in primary tumors and C70 is also found in metastases. It may become an effective target molecule for tumor immunotherapy.

### Summary of the invention

Objective of the invention: In view of the prior art described above, the present invention provides an Anti CD70 CAR-T cell and preparation method thereof and use thereof.

Technical solution: The present invention discloses an Anti CD70 CAR-T cell which expresses the SCFV (Anti CD70)-CD8^{™}-4-1BB-CD3ζ fusion protein in T lymphocytes. That is, the CAR-T cells of the present invention take CD70 domain as the target and express SCFV (Anti CD70)-CD8^{™}-4-1BB-CD3ζ fusion protein in T lymphocytes.

Wherein, the T lymphocytes are derived from PBMC isolated from human peripheral blood.

The amino acid sequence of the SCFV-CD8^{™}4-1BB-CD3ζ fusion protein is shown in SEQ ID NO. 7.

Preferably, in the SCFV-CD8^{™} 4-1BB-CD3ζ fusion protein, the amino acid sequence of CD8 Leader is as shown in SEQ ID NO. 1, and the amino acid sequence of CD70 SCFV (containing (G₄S)₃) is as shown in SEQ ID NO. 2.

Preferably, in the SCFV-CD8^{™} 4-1BB-CD3ζ fusion protein, the molecular sequence of CD8_Hinge is as shown in SEQ ID NO. 3, and the amino acid sequence of CD8^{™} is as shown in SEQ ID NO. 4.

Preferably, in the SCFV-CD8^{™} 4-1BB-CD3ζ fusion protein, the amino acid sequence of 4-1BB is shown as SEQ ID NO. 5, and the amino acid sequence of CD3ζ is shown as SEQ ID NO. 6.
SEQ ID NO. 1 (CD8 Leader sequence):
   MALPVTALLLPLALLLHAARP
SEQ ID NO.2 (CD70 Scfv): 
QVQLVQSGGGISWFPGGSIKISGSGFTSWFYNVWKQGPGKGGIQWVSDINNQGGTLFA
DSWKGKFTISKENSRNSIFIVNRSIKGYDTGWFYCAKDAGYSNHWPIFDSWGGGGSGS
GSGSGSGGSGGSGGSGGSGSGSNQTPGQGPKILIFNTNKGGGPDVKFSGSIIGNKGAGG
DDQAQFTWGISVPSW QFGAGTQITVLG
SEQ ID NO. 3 (CD8 Hinge):
   TTTPAPRPPTPAPTIASQPLSLRPEACRPAAGGAVHTRGLDFACDIY
SEQ ID NO. 4 (CD8_TM):
   IWAPLAGTCGVLLLSLVITLYC
SEQ ID NO. 5 (4-1BB):
   KRGRKKLLYIFKQPFMRPVQTTQEEDGCSCRFPEEEEGGCEL
SEQ ID NO. 6 (CD3 zeta): 
RVKFSRSADAPAYQQGQNQLYNELNLGRREEYDVLDKRRGRDPEMGGKPRRKNPQEG
LYNELQKDKMAEAYSEIGMKGERRRGKGHDGLYQGLSTATKDTYDALHMQALPPR
SEQ ID NO. 7:

MALPVTALLLPLALLLHAARPQVQLVQSGGGISWFPGGSIKISGSGFTSWFYNVWKQGP
GKGGIQWVSDINNQGGTLGFYCAKDAGYSNHWPIFDSWGGGGSGGSGGGSGGGGGG
SQPSITWSPGGGTWGSGGSNQTPGQGPKILIFNTNKKGAITIVGAQGEDDQAQFTWGIS
VPSWQFGAGTQITVLGTTTPAPRPPTPAPTIASQPLSLRPEACRPAAGAVHTRGLDFACDI
YIWAPLAGTCGLLLSLVITLYCKLLYIFKQPFMRPVQQSRSADAPAYQQLDLDKNPQEGL
YNELGKKGHDGLYQGLGSTATKDTYDALHMQALPPR

In the Anti CD70 CAR-T cell of the present application, the surface of the T cell expresses the molecule of the Anti CD70 SCFV sequence, and the 4-1BB-CD3ζ molecule transmits the T cell activation signal in the T cell.

The present invention also discloses a preparation method of the Anti CD70 CAR-T cell above, which comprises the following steps:
(1) Synthesis and amplification of SCFV-CD8^{™}-4-1BB-CD3ζfusion protein gene, and cloning the SCFV-CD8^{™}-4-1BB-CD3 Zeta Fusion Protein Gene into lentivirual expression vector;
(2) Infecting 293T cells with lentivirual packaging plasmids and lentivirual expression vector plasmids obtained in step (1), packaging and preparing lentivirus;
(3) Separating T lymphocytes, culturing and amplifying, infecting T lymphocytes with lentivirus obtained in step (2), and making the T lymphocytes express SCFV-CD8^{™}-4-1BB-CD3ζfusion protein to obtain the Anti CD70 CAR-T cells.

The present invention also discloses the use of the Anti CD70 CAR-T cells in preparing drugs for treating tumors with high expression of CD70 molecules. Further, the tumor is renal cancer.

Beneficial effects: the present invention uses CD70 antibody for the construction of CAR-T cells, and proposes that CD70 molecules are used as the target antigen. When the constructed CAR-T cells are in contact with tumor cells expressing CD70 molecules, the distance will be pulled closer, increasing T cell killing specificity; the prepared Anti CD70 CAR-T cells have more efficient tumor killing activity and can be used to prepare drugs for tumors with high expression of CD70 molecules.

### Description of the figures

Fig. 1 is a structure diagram of the lentivirual plasmid vector PLV-SCFV (anti CD70)-CD8^{™}-4-1BB-CD3ζof the present invention;
Fig. 2 is a diagram showing the flow detection results of renal cells (786-0) with high expression of CD70 antigen of the present invention;
Fig. 3 is a flow assay result diagram of the constructed Anti CD70 CAR-T cells of the present invention;
Fig. 4 is an *in vitro* killing effect diagram of the CAR-T of the present invention (786-0 is the experimental group and MCF-7 is the negative control group);
Fig. 5 is an *in vitro* killing effect diagram of CAR-T cells under different effector-target ratios of the present invention (measured by RTCA);
Fig. 6 is a graph showing the results of survival rate change of mice that obtained by animal level experiments of the present invention;
Fig. 7 is a graph showing the results of volume change of subcutaneous transplanted tumor in mice that obtained by animal level experiments of the present invention;
Fig. 8 is a graph showing the results of changes in body weight of mice during the experimental period that obtained by animal level experiments of the present invention.

### Detailed description

The technical solution in the present invention will be clearly and completely described combined with specific embodiments below.

### EXAMPLE 1: preparation of lentivirual expression vector

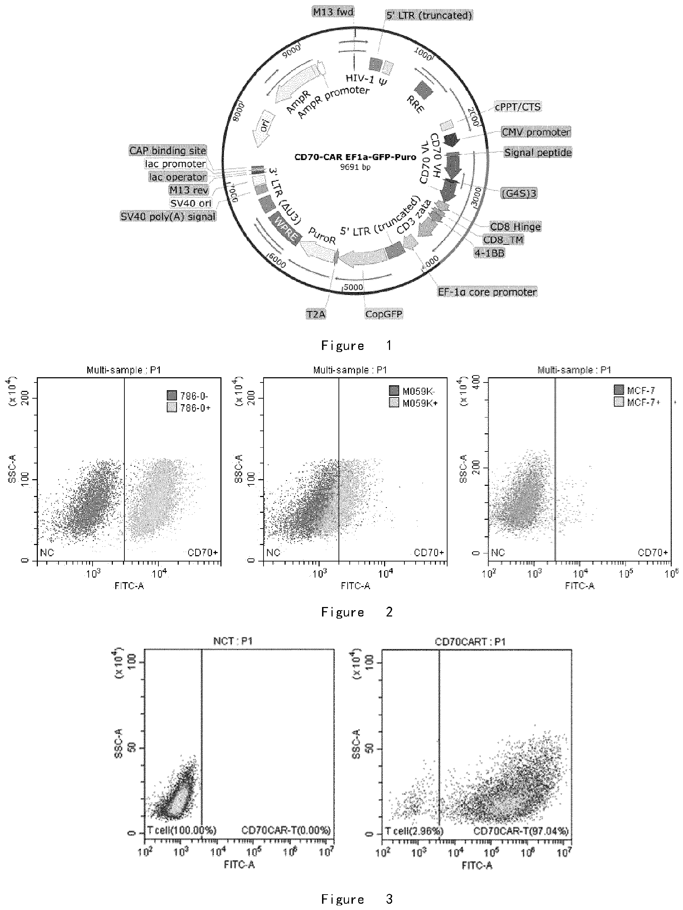

SCFV (anti CD70)-CD8TM-4-1BB-CD3ζ fusion gene sequence was gene synthesised. The gene sequence is shown in SEQ ID NO. 7, which is connected to the PLV vector by restriction digestion, and the upstream of the gene is the EP-1a promoter. The vector was transformed into DH5α *Escherichia coli* strain and the screening was performed by ampicillin to obtain the positive clone. The plasmid was extracted and the clone was digested to identify, then the PLV-SCFV (anti CD70)-CD8^{™}-4-1BB-CD3ζlentivirual packaging vector was obtained, see Fig. 1.

### EXAMPLE 2: preparation of lentivirus

24 hours before transfection, 293T cells were inoculated into T75 culture flasks at a rate of approximately 8×10⁶ per flask. Ensure that lentivirus packing is performed when the cells are evenly distributed in the culture flask with a fusion degree of about 80%.

Prepare plasmids and transfection reagent diluent
1. Mix PEI 40K transfection reagent evenly by vortex oscillation.
2. Prepare two centrifuge tubes, and prepare plasmids and transfection reagent diluent in the following order.

| Centrifuge tube 1 (plasmid DNA) | Centrifuge tube 2 (transfection reagent) |
|---|---|
| Lentivirual vector 10 µ g | PEI 40K transfection reagent 40 µ l |
| VSVG vector 7.5 µ g | DMEM serum-free medium 460 µ l |
| G-Puro vector 2.5 µ g | Total volume 500 µ l |
| DMEM serum-free medium X µ l | |
| Total volume 500 µ l | |

3. Mix well.
4. Add the diluent of transfection reagent (centrifuge tube 2) to the plasmid DNA solution (centrifuge tube 1), and immediately mix well. It is important to note the order of addition.
5. Incubate the transfection mixture at room temperature for 15~20 minutes.
6. Add each 1ml of transfection mixture to the 293T cell culture flask, and gently pipette the medium to mix.
7. Incubate at 37°C for 6 hours.
8. Remove the culture medium containing transfection reagent, and change the medium with 20 ml virus culture medium.
9. Collect cell culture supernatant 48 hours after transfection. 500g centrifuge for 10min to remove cell debris. The supernatant can be directly used for lentivirus infection, and can also be used for virus titer determination or virus concentration. For long time storage, it can be cryopreserved at -80 °C.

### EXAMPLE 3: Preparation of Anti CD70 CAR-T cells

0.5 ml blood was taken for rapid detection of pathogenic microorganisms, and microbial infections such as HBV, HCV, HDV, HEV, HIV-1/2, treponema pallidum and parasites were excluded. Peripheral blood mononuclear cells (PBMC) were collected by apheresis machine. Complete growth medium was prepared, 5% autologous AB or FBS was added to PBS, with IL-2 at the concentration of 20 ng/ml. The separated PBMC was diluted with medium to 2 × 10/ml, and 50u1 was taken to detect the purity of T cells in PBMC by flow cytometry. Day 0, buffer 1 was configured, and 1% FBS was added to PBS. Beads were oscillated for 30s or shaken up and down for 5min manually. CD3/CD8 beads were taken out in 1.5 ml EP tube according to the ratio of beads to T cells of 3:1. 1ml Buffer1 was added to wash beads, then beadslmin was sucked out of EP tube with magnet, and washing solution was discarded. Repeat twice. Then beads were suspended to original volume with culture medium. Cells and beads were mixed and added to the appropriate culture flask at 2 × 10⁵ PBMC/ml. The cell density was adjusted to 2 × 10⁵/ml the next day, and virus vectors were added according to the ratio of virus vector: cell = 1: 5. 4ug/ml of polybrene and 20ng/ml IL-2 were added at the same time. After 4 hours, the cell density was adjusted to 5 × 10⁴/ml by adding fresh and complete culture medium. All the cells were centrifuged,and fresh culture medium was added to continue culture. The cell density was maintained at 0.5-1 × 10%/ml by changing the medium half every 2-3 days. After 10-12 days, the number of cells reached a level of 10⁶. The immune cells were obtained by centrifuged at 400g for 5min, and then washed twice with precooled PBS (400g, 5min). The cell population and CAR-T cell ratio were detected by blood cell counting plate and flow cytometry, as shown in Fig. 3. According to the illustration of Fig. 3, the positive rate of CAR-T cells prepared by the method of this example is over 97%. The color change of the culture medium, cell density and cell morphology were observed every day and recorded accordingly. In the process of gradually expanding culture, IL-2 needed for the total volume was added.

### EXAMPLE 4: Screening and detection of engineering cell lines

(1) Screening cancer cell lines with high expression of CD70 for culture;
(2) Each 20,000 different cells were taken, centrifuged at 400g, 5min, and washed twice with precooled PBS. Then 2 µ1 of CD70 antibody (BD) was added respectively to incubate for 20min in the dark. After centrifugation, the cells were washed with precooled PBS for one time, and the cells were re-suspended with 200 µ1 PBS. The expression of CD70 was detected by flow cytometry (see Fig. 2). The experimental results verified that renal cell line 786-0 highly expresses CD70 (closed to 100%), and glioma cell line MO59K also has a high expression rate, which can be used as a target cell for subsequent killing experiment. Breast cancer cell line MCF-7 almost does not express CD70 and can be used as a negative control.

### EXAMPLE 5

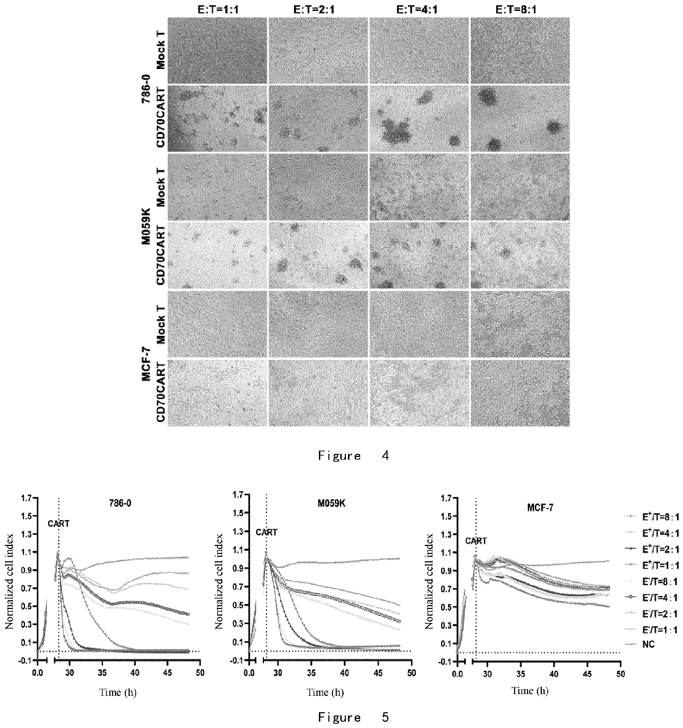

The killing effect of 786-0 and M059K mediated by CD70 CAR-T cell was monitored by RTCA in real time (MCF-7 was used as a negative control)
1. Before inoculation of target cells, 100 µ1 1640 medium was added to each well and the plate was put into RTCA. The value at this time was recorded as "0";
2. Each well was added with 1×10⁵ target cells, and they were incubated at 37°C with 5% CO₂ for 24 hours until the cell confluence reached over 90%;
3. The 96-well plate was taken out and 200 µ1 CD70 CAR-T cells were added to each well according to E/T ratio of 1: 1, 2: 1, 4: 1 and 8: 1.
4. The electrode plate was put back into RTCA and fasten, and continued to culture;
5. After 48 hours, the experiment was finished, and the cell killing results were observed under microscope, as shown in Fig. 4. According to the illustration, the killing effect of CD70 CAR-T on renal cell carcinoma cells (786-0) and glioma (M059K) increases with the increase of the effector-target ratio, while no killing effect is found on cells that do not express CD70 (MCF-7) in different effector-target ratios.
6. Analyzing the data, the results are as shown in Fig. 5. According to the illustration, it can be seen that the time required for CD70 CAR-T to achieve the same killing effect on renal cell cells (786-0) and glioma cells (M059K) is shortened with the increase of the effector-target ratio, and the cancer cells can be almost eliminated by different effector-target ratios, while no killing effect is found on breast cancer cells (MCF-7) without CD70 expression in different effector-target ratios.

### EXAMPLE 6

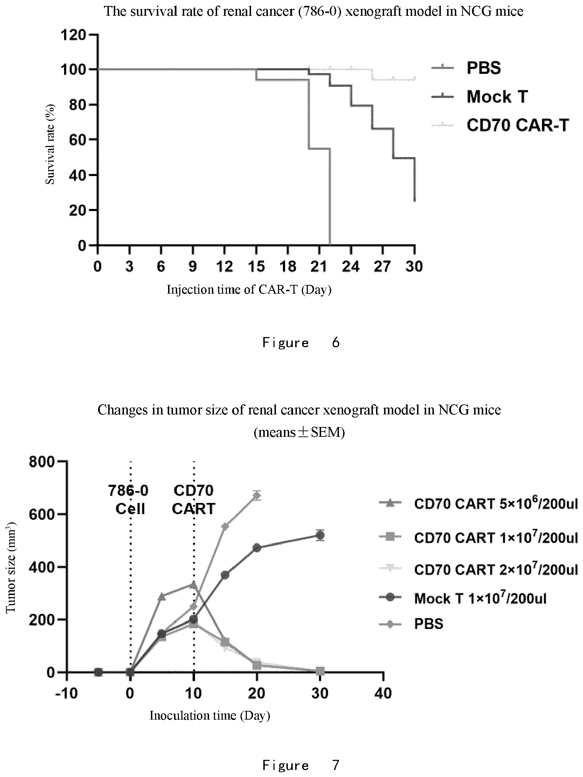

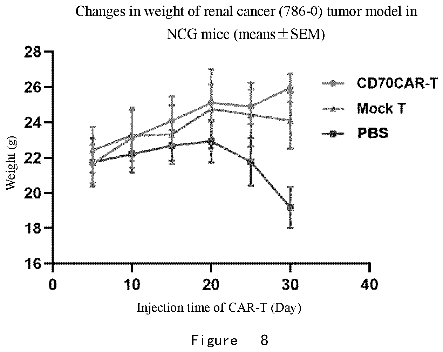

NCG mice (purchased from Biocytogen Co., Ltd.) were subcutaneously inoculated with human renal cell carcinoma cell 786-0 with high expression of CD70 on the left and right flanks of their backs to establish the subcutaneous transplanted tumor model. After 20 and 25 days after tumor injection, mice were divided into groups (5 mice in each group) and treated with Anti CD70 CAR-T cells, mock T cells and PBS via tail vein infusion. The survival of mice was observed and recorded every three days. The results are as shown in Fig. 6. According to the illustration of Fig. 6, the remission rate of mice injected with CD70 CAR-T group was 94%, that of mice injected with mock T group was 25%, and all mice injected with PBS group died on the 22nd day. The tumor size was measured every five days. The results are shown in Fig. 7. According to the illustration, the subcutaneous transplanted tumors of mice injected with three different doses of CD70 CAR-T cells disappeared 20 days after injection of CD70 CAR-T cells, and the subcutaneous transplanted tumors of mice injected with mock T group reached an average of 510 mm², while the subcutaneous transplanted tumor of mice injected with PBS reached an average of 700 mm². The weight changes of mice were recorded. The results are shown in Fig. 8. According to the illustration, the weight of mice injected with CD70 CAR-T and mock T increased, while the weight of mice injected with PBS decreased from 21g to 19g on average. All the above results show that CD70 CAR-T has a good effect on tumor inhibition, and the mortality and survival status of transplanted tumor mice are well improved.

## Claims

1. An Anti CD70 CAR-T cell, wherein the CAR-T cell targets the CD70 domain and expresses SCFV-CD8^{™}-4-1BB-CD3ζ fusion protein in T lymphocytes.

2. The Anti CD70 CAR-T cell of claim 1, wherein the T lymphocyte is derived from PBMC isolated from human peripheral blood.

3. The Anti CD70 CAR-T cell of claim 1, wherein the amino acid sequence of the SCFV-CD8^{™}4-1BB-CD3ζ fusion protein is as shown in SEQ ID NO. 7.

4. The Anti CD70 CAR-T cell of claim 1, wherein in the SCFV-CD8^{™}4-1BB-CD3ζfusion protein, the amino acid sequence of CD8 Leader is as shown in SEQ ID NO. 1, and the amino acid sequence of CD70 SCFV (containing (G₄S)₃) is as shown in SEQ ID NO. 2.

5. The Anti CD70 CAR-T cell of claim 1, wherein in the SCFV-CD8^{™}4-1BB-CD3ζfusion protein, the amino acid sequence of CD8_Hinge is as shown in SEQ ID NO. 3, and the amino acid sequence of CD8^{™} is as shown in SEQ ID NO. 4.

6. The Anti CD70 CAR-T cell of claim 1, wherein in the SCFV-CD8^{™}4-1BB-CD3ζfusion protein, the amino acid sequence of 4-1BB is shown as SEQ ID NO. 5, and the amino acid sequence of CD3 zeta is as shown in SEQ ID NO. 6.

7. The Anti CD70 CAR-T cell of claim 1, wherein the molecule of the Anti CD70 SCFV sequence is expressed on the surface of the T cell, and a T cell activation signal is transmitted intracellular of the T cell by the 4-1BB-CD3ζmolecule.

8. The method for preparing the Anti CD70 CAR-T cells of any one of claims 1-7, which comprises the following steps:
(1) Synthesis and amplification of SCFV-CD8^{™}-4-1BB-CD3ζfusion protein gene, and cloning the SCFV-CD8^{™}-4-1BB-CD3ζfusion protein gene into lentivirual expression vector;
(2) Infecting 293T cells with lentivirual packaging plasmid and lentivirual expression vector plasmid obtained in step (1), packaging and preparing lentivirus;
(3) Separating T lymphocytes, culturing and amplifying, infecting T lymphocytes with lentivirus obtained in step (2), and making the T lymphocytes express SCFV-CD8^{™}-4-1BB-CD3ζfusion protein to obtain the Anti CD70 CAR-T cells.

9. Use of the Anti CD70 CAR-T cell of any one of claims 1-7 in the preparation of a drug for the treatment of a tumor with high expression of CD70 molecules.

10. The use of claim 9, wherein the tumor is renal cell carcinoma or brain glioma.

## Amended claims

### Amended claims under Art. 19.1 PCT

1. An Anti CD70 CAR-T cell, wherein the CAR-T cell targets the CD70 domain and expresses SCFV-CD8^{™}-4-1BB-CD3ζ fusion protein in T lymphocytes; wherein the T lymphocyte is derived from PBMC isolated from human peripheral blood; wherein the amino acid sequence of the SCFV-CD8^{™}4-1BB-CD3ζ fusion protein is as shown in SEQ ID NO. 7.

2. The method for preparing the Anti CD70 CAR-T cells of any one of claims 1, which comprises the following steps:
(1) Synthesis and amplification of SCFV-CD8^{™}-4-1BB-CD3ζfusion protein gene, and cloning the SCFV-CD8^{™}-4-1BB-CD3ζfusion protein gene into lentivirual expression vector;
(2) Infecting 293T cells with lentivirual packaging plasmid and lentivirual expression vector plasmid obtained in step (1), packaging and preparing lentivirus;
(3) Separating T lymphocytes, culturing and amplifying, infecting T lymphocytes with lentivirus obtained in step (2), and making the T lymphocytes express SCFV-CD8^{™}-4-1BB-CD3ζfusion protein to obtain the Anti CD70 CAR-T cells.

3. Use of the Anti CD70 CAR-T cell of any one of claims 1-7 in the preparation of a drug for the treatment of a tumor with high expression of CD70 molecules; wherein the tumor is renal cell carcinoma or brain glioma.


### SEQUENCE LISTING

<110>  ZHEJIANG BLUE SHIELD PHARMACEUTICAL CO., LTD.

<120> Anti CD70 CAR-T cell, and preparation method thereof and use thereof

<130>  X7-B-65270 EP

<140>  PCT/CN2020/092192
<141>  2020-05-26

<150>  CN201910857176.1
<151>  2019-09-11

<160>  7

<170>  SIPOSequenceListing 1.0

<210>  1
<211>  21
<212>  PRT
<213>  Artificial Sequence

<220>
<223>  CD8 Leader sequence

<400>  1
Met Ala Leu Pro Val Thr Ala Leu Leu Leu Pro Leu Ala Leu Leu Leu
1               5                   10                  15
His Ala Ala Arg Pro
            20

<210>  2
<211>  245
<212>  PRT
<213>  Artificial Sequence

<220>
<223>  CD70 SCFV

<400>  2
Gln Val Gln Leu Val Gln Ser Gly Gly Gly Ile Ser Trp Phe Pro Gly
1               5                   10                  15
Gly Ser Ile Lys Ile Ser Gly Ser Gly Phe Thr Phe Ser Trp Phe Tyr
            20                  25                  30
Asn Val Trp Lys Gln Gly Pro Gly Lys Gly Ile Gln Trp Val Ser Asp
        35                  40                  45
Ile Asn Asn Gln Gly Gly Thr Leu Phe Val Ala Asp Ser Trp Lys Gly
    50                  55                  60
Lys Phe Thr Ile Ser Lys Glu Asn Ser Arg Asn Ser Ile Phe Ile Val
65                  70                  75                  80
Asn Arg Ser Ile Lys Gly Tyr Asp Thr Gly Trp Phe Tyr Cys Ala Lys
                85                  90                  95
Asp Ala Gly Tyr Ser Asn His Trp Pro Ile Phe Asp Ser Trp Gly Gln
            100                 105                 110
Gly Thr Ile Trp Thr Val Ser Ser Gly Gly Gly Gly Ser Gly Gly Gly
        115                 120                 125
Gly Ser Gly Gly Gly Gly Ser Gln Gly Val Trp Ser Asn Gln Pro Ser
    130                 135                 140
Ile Thr Trp Ser Pro Gly Gly Thr Trp Thr Ile Leu His Cys Gly Ile
145                 150                 155                 160
Lys Ser Gly Ser Ser Asp Asn Phe Pro Thr Trp Phe Asn Gln Thr Pro
                165                 170                 175

Gly Gln Gly Pro Lys Ile Leu Ile Phe Asn Thr Asn Thr Lys His Ser
            180                 185                 190
Gly Trp Pro Asp Val Lys Phe Ser Gly Ser Ile Ile Gly Asn Lys Gly
        195                 200                 205
Ala Ile Thr Ile Val Gly Ala Gln Gly Glu Asp Asp Gln Ala Gln Phe
    210                 215                 220
Thr Trp Gly Ile Ser Val Pro Ser Trp Gln Phe Gly Ala Gly Thr Gln
225                 230                 235                 240
Ile Thr Val Leu Gly
                245

<210>  3
<211>  47
<212>  PRT
<213>  Artificial Sequence

<220>
<223>  CD8_Hinge

<400>  3
Thr Thr Thr Pro Ala Pro Arg Pro Pro Thr Pro Ala Pro Thr Ile Ala
1               5                   10                  15
Ser Gln Pro Leu Ser Leu Arg Pro Glu Ala Cys Arg Pro Ala Ala Gly
            20                  25                  30
Gly Ala Val His Thr Arg Gly Leu Asp Phe Ala Cys Asp Ile Tyr
        35                  40                  45

<210>  4
<211>  22
<212>  PRT
<213>  Artificial Sequence

<220>
<223>  CD8_TM

<400>  4
Ile Trp Ala Pro Leu Ala Gly Thr Cys Gly Val Leu Leu Leu Ser Leu
1               5                   10                  15
Val Ile Thr Leu Tyr Cys
            20

<210>  5
<211>  42
<212>  PRT
<213>  Artificial Sequence

<220>
<223>  4-1BB

<400>  5
Lys Arg Gly Arg Lys Lys Leu Leu Tyr Ile Phe Lys Gln Pro Phe Met
1               5                   10                  15
Arg Pro Val Gln Thr Thr Gln Glu Glu Asp Gly Cys Ser Cys Arg Phe
            20                  25                  30
Pro Glu Glu Glu Glu Gly Gly Cys Glu Leu
        35                  40

<210>  6
<211>  112
<212>  PRT
<213>  Artificial Sequence

<220>

<223>  CD3 zeta

<400>  6
Arg Val Lys Phe Ser Arg Ser Ala Asp Ala Pro Ala Tyr Gln Gln Gly
1               5                   10                  15
Gln Asn Gln Leu Tyr Asn Glu Leu Asn Leu Gly Arg Arg Glu Glu Tyr
            20                  25                  30
Asp Val Leu Asp Lys Arg Arg Gly Arg Asp Pro Glu Met Gly Gly Lys
        35                  40                  45
Pro Arg Arg Lys Asn Pro Gln Glu Gly Leu Tyr Asn Glu Leu Gln Lys
    50                  55                  60
Asp Lys Met Ala Glu Ala Tyr Ser Glu Ile Gly Met Lys Gly Glu Arg
65                  70                  75                  80
Arg Arg Gly Lys Gly His Asp Gly Leu Tyr Gln Gly Leu Ser Thr Ala
                85                  90                  95
Thr Lys Asp Thr Tyr Asp Ala Leu His Met Gln Ala Leu Pro Pro Arg
            100                 105                 110

<210>  7
<211>  489
<212>  PRT
<213>  Artificial Sequence

<220>
<223>  SCFV-CD8 TM4-1BB-CD3 zeta

<400>  7
Met Ala Leu Pro Val Thr Ala Leu Leu Leu Pro Leu Ala Leu Leu Leu
1               5                   10                  15
His Ala Ala Arg Pro Gln Val Gln Leu Val Gln Ser Gly Gly Gly Ile
            20                  25                  30
Ser Trp Phe Pro Gly Gly Ser Ile Lys Ile Ser Gly Ser Gly Phe Thr
        35                  40                  45
Phe Ser Trp Phe Tyr Asn Val Trp Lys Gln Gly Pro Gly Lys Gly Ile
    50                  55                  60
Gln Trp Val Ser Asp Ile Asn Asn Gln Gly Gly Thr Leu Phe Val Ala
65                  70                  75                  80
Asp Ser Trp Lys Gly Lys Phe Thr Ile Ser Lys Glu Asn Ser Arg Asn
                85                  90                  95
Ser Ile Phe Ile Val Asn Arg Ser Ile Lys Gly Tyr Asp Thr Gly Trp
            100                 105                 110
Phe Tyr Cys Ala Lys Asp Ala Gly Tyr Ser Asn His Trp Pro Ile Phe
        115                 120                 125
Asp Ser Trp Gly Gln Gly Thr Ile Trp Thr Val Ser Ser Gly Gly Gly
    130                 135                 140
Gly Ser Gly Gly Gly Gly Ser Gly Gly Gly Gly Ser Gln Gly Val Trp
145                 150                 155                 160
Ser Asn Gln Pro Ser Ile Thr Trp Ser Pro Gly Gly Thr Trp Thr Ile
                165                 170                 175
Leu His Cys Gly Ile Lys Ser Gly Ser Ser Asp Asn Phe Pro Thr Trp
            180                 185                 190
Phe Asn Gln Thr Pro Gly Gln Gly Pro Lys Ile Leu Ile Phe Asn Thr
        195                 200                 205
Asn Thr Lys His Ser Gly Trp Pro Asp Val Lys Phe Ser Gly Ser Ile
    210                 215                 220
Ile Gly Asn Lys Gly Ala Ile Thr Ile Val Gly Ala Gln Gly Glu Asp
225                 230                 235                 240
Asp Gln Ala Gln Phe Thr Trp Gly Ile Ser Val Pro Ser Trp Gln Phe
                245                 250                 255
Gly Ala Gly Thr Gln Ile Thr Val Leu Gly Thr Thr Thr Pro Ala Pro
            260                 265                 270
Arg Pro Pro Thr Pro Ala Pro Thr Ile Ala Ser Gln Pro Leu Ser Leu
        275                 280                 285

Arg Pro Glu Ala Cys Arg Pro Ala Ala Gly Gly Ala Val His Thr Arg
    290                 295                 300
Gly Leu Asp Phe Ala Cys Asp Ile Tyr Ile Trp Ala Pro Leu Ala Gly
305                 310                 315                 320
Thr Cys Gly Val Leu Leu Leu Ser Leu Val Ile Thr Leu Tyr Cys Lys
                325                 330                 335
Arg Gly Arg Lys Lys Leu Leu Tyr Ile Phe Lys Gln Pro Phe Met Arg
            340                 345                 350
Pro Val Gln Thr Thr Gln Glu Glu Asp Gly Cys Ser Cys Arg Phe Pro
        355                 360                 365
Glu Glu Glu Glu Gly Gly Cys Glu Leu Arg Val Lys Phe Ser Arg Ser
    370                 375                 380
Ala Asp Ala Pro Ala Tyr Gln Gln Gly Gln Asn Gln Leu Tyr Asn Glu
385                 390                 395                 400
Leu Asn Leu Gly Arg Arg Glu Glu Tyr Asp Val Leu Asp Lys Arg Arg
                405                 410                 415
Gly Arg Asp Pro Glu Met Gly Gly Lys Pro Arg Arg Lys Asn Pro Gln
            420                 425                 430
Glu Gly Leu Tyr Asn Glu Leu Gln Lys Asp Lys Met Ala Glu Ala Tyr
        435                 440                 445
Ser Glu Ile Gly Met Lys Gly Glu Arg Arg Arg Gly Lys Gly His Asp
    450                 455                 460
Gly Leu Tyr Gln Gly Leu Ser Thr Ala Thr Lys Asp Thr Tyr Asp Ala
465                 470                 475                 480
Leu His Met Gln Ala Leu Pro Pro Arg
                485